Europäisches Patentamt

European Patent Office

Office européen des brevets

(11) Publication number: **0 325 014**
**A1**

## (12) EUROPEAN PATENT APPLICATION

(21) Application number: 88300460.8

(22) Date of filing: 20.01.88

(51) Int. Cl.⁴: **C07D 211/74**

(43) Date of publication of application:
26.07.89 Bulletin 89/30

(84) Designated Contracting States:
**AT BE CH DE ES FR GB GR IT LI LU NL SE**

(71) Applicant: **ICI AMERICAS INC**
**Concord Pike & New Murphy Road**
**Wilmington Delaware 19897(US)**

(72) Inventor: **Kruse, Walter M.**
**1 Woodbury Court**
**Wilmington, DE 19805(US)**
Inventor: **Stephen, John F.**
**200 William Penn Blvd.**
**West Chester, PA 19380(US)**

(74) Representative: **James, David Gomer et al**
**Imperial Chemical Industries PLC Legal**
**Department: Patents Po Box 6 Bessemer**
**Road**
**Welwyn Garden City Herts, AL7 1HD(GB)**

(54) Process for the preparation of 2,2,6,6-tetramethyl-4-oxopiperidine.

(57) 2,2,6,6-tetramethyl-4-oxopiperidine is prepared from acetone and ammonia in the presence of catalytic amounts of active halogen compounds selected from sulfonylhalides, sulfurylhalides, N-haloamides, N-haloimides, $\beta$-haloesters, $\alpha$-haloketones, $\alpha$-halohydroxy compounds, and $\beta$-halonitriles.

EP 0 325 014 A1

# PROCESS FOR THE PREPARATION OF 2,2,6,6-TETRAMETHYL-4-OXOPIPERIDINE

The present invention is directed to an improved process for synthesis of 2,2,6,6-tetramethyl-4-oxopiperidine. It is directed to a process for producing 2.2.6,6-tetramethyl-4-oxopiperidine directly from acetone and ammonia at low temperatures and pressures. Specifically it is directed to reacting acetone with ammonia in the presence of novel catalysts. Such catalysts are selected from compounds with active halogens selected from alkyl and aryl derivatives of sulfonylhalides, haloamides, haloimides, β-haloesters, β-halonitriles, α-halohydroxy compounds and β-haloketones and sulfurylhalides.

Conventional synthesis for the preparation of 2,2,6,6-tetramethyl-4-oxypiperdine(triacetonamine) involves the reaction of excess acetone with ammonia in the presence of a Lewis acid at low temperatures to form acetonine as an intermediate which is thereafter hydrolyzed at higher temperatures. Acetonine is formed in the first step at room temperature and transformed to triacetonamine at 50-70° C. Triacetonamine is isolated by distillation in up to 85% yields or by precipitation with water as a hydrate in up to 73% yields.

We have now found that a group of active halogen compounds can be used in place of prior art catalysts to improve the reaction speed and increase yields at lower temperatures and pressures. The process comprises two stages which can be performed one immediately after the other in the same reactor so that it is particularly suitable for large scale commercial manufacture of a tetramethylpiperidine product. The process according to the invention is carried out as follows:

    a. Acetone is reacted with ammonia at concentrations of (0.5-1.5 mol NH₃ per mol acetone) in the presence of 0.01-0.1 mol (of an active halogen catalyst) per mol of acetone used at 5° to 30° C, and thereafter

    b. with additional acetone in concentrations of (5-10 mols acetone per mol NH₃) by further heating at 50°-70° C.

As catalysts may be employed: sulfonyl halides $R-SO_2X$; sulfuryl halides $SO_2X_2$; N-haloimides $RCO-N(X)-COR$; N-haloamides $RCO-(NHX)$; β-haloesters $RCH(X)$ $(CH_2)-CO_2R$; β-halonitriles $RCH(X)CH_2-CN$; α-haloalcohols $RC-(OH)-CH_2X$; and α-haloketones $RCO-CH_2(X)$ wherein X is chlorine or bromine and R is selected from hydrocarbyl radicals selected from alkyl, aralkyl, alkylaryl groups having up to 22 carbon atoms. Such compounds include tetrachloro-1,4-benzoquinone, methanesulfonylchloride, sulfurylchloride. 1,3-dibromo-5,5-dimethylhydantoin, 1,3-dichloro-5,5-dimethylhydantion, N-bromosuccinimide, trichloromelamine, trichloroisocyanuric acid, 3-chloropropionitrile, ethyl-3-chloropropionate, 1,3-dichloro-2-propanol, and N-chlorsuccinimide and the chloro or bromo equivalents.

The first stage of the reaction is carried out at temperatures of about 25-30° C at atmospheric pressure. The second stage is then carried out with the addition of acetone at temperatures of 50-70° C.

The practice of the process of the invention can better be understood by reference to the following non-limiting examples wherein all proportions are expressed in parts by weight unless otherwise specified.

## Example 1

Into a one liter bottle provided with rubber liner, steel crown cap and magnetic stirrer were placed 7.4 g (0.0375 mol) 1,3-dichloro-5,5-dimethylhydantion and 135 g acetone. The crown cap was equipped with a small opening through which a needle was inserted for evacuation or injection of liquids. A water aspirator vacuum was applied for a few minutes and the bottled weighed. After evacuations 21 g ammonia was introduced within 1 hour at 20° C. After an additional 2 hours at room temperature 300 g acetone was added and the temperature raised to 67° C for 4 hours and continued to react over night at 56° C. According to gas chromatographic determination the reaction solution contained 138 g, 2,2,6,6-tetramethyl-4-oxopiperidine-hydrate. To this solution was added 20 g sodium hydroxide and stirred for 1 hour. The two phases were separated and 270 g acetone and low boiling by products were recovered by fractional distillation at 30-84° C under 15 mm Hg pressure to recover acetone, mesityl oxide, diacetone alcohol acetonine and a few grams of triacetonamine. In another cut (boiling point 84° - 88° C at 15 mm Hg) the product was recovered for a yield of 84.2% (based on used acetone).

## Example 2

According to a procedure and equipment outlined for Example 1, 10.7 g (0.0375 mol) 1,3-dibromo-5,5-dimethylhydantion and 135 g acetone were placed into the 1 liter bottle. 20.5 g ammonia were taken up within 3 hours. After addition of 300 g acetone, reaction mixture was kept at 67°C for 8 hours. The yield determined by gas chromatographic techniques was determined to be 78.1% (based on used acetone).

## Example 3

According to the procedure outlined for Example 1, 4.3 g (0.0375 mol) methanesulfonylchloride and 135 g acetone and 21 g ammonia was added to the reactor. The reaction was continued at room temperature for 1.5 hours. After addition of 300 g acetone reaction was continued at 56°C for 16 hours. The yield determined by gas chromatographic technique was 81.5% (based on used acetone).

## Example 4

According to the procedure of Example 1, 4.3 g (0.0375 mol) methanesulfonylchloride and 435 g (7.5 mol) acetone and 21 g ammonia were introduces into the reactor and placed in a water bath at 69°C and kept there for 10 hours. After the similar recovery procedure of Example 1 the yield of 80.8% (based on used acetone) was obtained.

## Example 5

Employing the procedure of Example 1, 13.3 g (0.075 mol) N-bromosuccinimide gave a yield of 83.5% (based on used acetone).

## Example 6

According to the procedure of Example 1, 10.2 g (0.075 mol) ethyl-3-chloroproprionate and 21 g ammonia gave a yield of 81.8% (based on used acetone).

## Example 7

According to the procedure of Example 1, 7.0 g (0.075 mol) 3-chloropropionitrile and 22 g ammonia produced a yield of 79.7% (based on used acetone).

## Example 8

According to the procedure of Example 1, 3 g (0.045 mol) sulfurylchloride and 21 g ammonia produced a yield of 84.4% (based on used acetone).

**Claims**

1. In a process for the preparation of 2,2,6,6-tetramethyl-4-oxopiperidine which comprises reacting acetone with ammonia at temperatures ranging from 5°-70°C the improvement which comprises carrying out said reaction in the presence of catalytic amounts of active halogen compounds selected from those in the group consisting of $SO_2X_2$, $R\text{-}SO_2X$, $RCO\text{-}N(X)\text{-}COR$, $RCO(NHX)$, $RCH(X)CH_2\text{-}CO_2R$, $RCH(X)CH_2\text{-}CN$, $RC(OH)\text{-}CH_2X$, and $RCO\text{-}CH_2(X)$ wherein X is chlorine or bromine and R is a hydrocarbyl group selected from alkyl, aryl, arylalkyl, and alkylaryl groups having up to 22 carbon atoms.

2. The process of Claim 1 wherein acetone is reacted with ammonia in concentrations of 0.5-1.0 mol $NH_3$ per mol acetone at temperatures in the range of 5-30°C, and thereafter the additional acetone in concentrations of 5-10 mols acetone per mol $NH_3$ by further heating at 50-70°C.

3. A process of Claim 1 wherein said active halogen compounds are selected from the group consisting of tetrachloro-1,4-benzoquinone, methanesulfonylchloride, sulfurylchloride, 1,3-dibromo-5,-5-dimethylhydantion, 1,3-dichloro-5,5-dimethylhydantion, N-bromosuccinimide, trichloromelamine, trichloroisocyanuric acid, 3-chloropropionitrile, ethyl-3-chloropropionate, 1,3-dichloro-2-propanol, and N-chlorosuccinimide and their chloro or bromo equivalents.

# EUROPEAN SEARCH REPORT

European Patent
Office

Application Number

EP 88 30 0460

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 037 548 (HOECHST AG) <br> * Whole document * | 1-3 | C 07 D 211/74 |
| Y | EP-A-0 152 935 (ADEKA ARGUS CHEMICAL CO., LTD) <br> * Claims 1-3 * | 1-3 | |
| Y | EP-A-0 152 934 (ADEKA ARGUS CHEMICAL CO., LTD) <br> * Whole document * | 1-3 | |
| Y | EP-A-0 074 607 (ADEKA ARGUS CHEMICAL CO., LTD) <br> * Whole document * | 1-3 | |
| Y | FR-A-2 455 038 (SA ARGUS CHEMICAL NV) <br> * Pages 3-4 * | 1-3 | |
| Y | DE-A-3 008 536 (HOECHST AG) <br> * Whole document * | 1-3 | |
| A | EP-A-0 013 865 (CIBA-GEIGY AG) <br> * Whole document * | 1-3 | TECHNICAL FIELDS SEARCHED (Int. Cl.4) <br><br> C 07 D 211/00 |
| A | GB-A-2 017 687 (CHIMOSA CHEMICA ORGANICA S.p.A.) <br> * Whole document * | 1-3 | |
| A | EP-A-0 004 104 (CHEMISCHE WERKE HULS AG) <br> * Pages 2-3 * | 1-3 | |

The present search report has been drawn up for all claims

| Place of search | Date of completion of the search | Examiner |
|---|---|---|
| THE HAGUE | 26-09-1988 | MAISONNEUVE J.A. |

EPO FORM 1503 03.82 (P0401)